# EUROPEAN PATENT SPECIFICATION

(11) **EP 0 729 518 B1**
(45) Date of publication and mention of the grant of the patent: **28.11.2001**
(21) Application number: 95929892.8
(22) Date of filing: 18.08.1995
(51) Int. Cl.: C12Q 1/70, C12Q 1/68

(54) **PRIMERS AND PROBES FOR THE AMPLIFICATION AND DETECTION OF CMV NUCLEIC ACID**
PRIMER UND SONDEN ZUR AMPLIFIZIERUNG UND ZUM NACHWEIS VON CMV NUKLEINSÄURE
AMORCES ET SONDES DESTINEES A L'AMPLIFICATION ET A LA DETECTION DE L'ACIDE NUCLEIQUE DU CYTOMEGALOVIRUS

(30) Priority: 18.08.1994 EP 94202360
(43) Date of publication of application: 04.09.1996
(73) Proprietor: Akzo Nobel N.V., 6824 BM Arnhem (NL)
(72) Inventor: SILLEKENS, Peter, Theodorus, Gerardus, NL-5291 ND Gemonde (NL); TIMMERMANS, Eveline, Catharina, Anna, Clasina, NL-5087 AM Diessen (NL)
(74) Representative: Van Gent, Marieke
(86) International application number: EP9503295
(87) International publication number: WO9606191

(56) References cited:
- EP-A- 0 329 822
- EP-A- 0 586 011
- WO-A-93/13227
- MOL CELL PROBES, (1994 AUG) 8 (4) 261-71, MEYER, T. ET AL. 'Identification of active cytomegalovirus infection by analysis of immediate-early, early and late transcripts in peripheral blood cells of immunodeficient patients.' cited in the application

## Description

The present invention is concerned with oligonucleotides that can be used as primers and probes in the detection of human Cytomegalovirus (HCMV) mRNA. Furthermore a method for the diagnosis of HCMV disease is provided.

Human Cytomegalovirus is an ubiquitous Herpes-type virus, having a double stranded DNA genome of about 240,000 nucleotides in length that infects 40-80% of humans before puberty. A prominent feature common to all herpesviruses is their establishment of lifelong persistence after infection and their ability to cause recurrent infection after reactivation (Stevens, J.G.. Microbiol. Rev. 53, 318-332., 1989). HCMV also becomes latent after primary infection which often occurs without clinical symptoms. Even recurrent infection in most cases goes asymptomatic or leads to only mild disease in the immunocompetent host. However, in congenitally infected infants and immunocompromised patients, such as allograft recipients (Meyers. J.D., *et al*., J. Infect. Dis. 153, 478-488., 1986) or AIDS patients (Drew, W.L. J Infect. Dis 158, 449-456., 1988; Drew, W.L. Clin. Infect. Dis 14, 608-615., 1992), where the fine balance between the immune system and the latently existing virus is disturbed, HCMV may cause severe and sometimes life-threatening disease, including retinitis, gastrointestinal disorders, and encephalitis (Drew, 1992). Early administration of antiviral drugs like ganciclovir and foscarnet can have significant beneficial effects on the prognosis of a patient (Jahn, G. *et al*., Intervirology 35, 60-72., 1993; Schmidt, G.M. *et al*., N. Engl. J Med. 324, 1005-1011., 1991). Therefore, with the availability of clinically effective antiviral therapy, early and sensitive diagnosis is of significant importance.

CMV specific antibodies, in particular IgM antibodies, can be used as a marker for CMV infection, but are of limited value when it comes to discrimination between latent and active infections. Most viral detection methods currently employed do not unambiguously allow for prediction of whether a given infection will be symptomatic. Furthermore serological methods are indirect and often lack sensitivity. Viral culture is a more direct diagnostic parameter for CMV viremia. Although CMV culture from blood cells appeared to be indicative for an active CMV infection, the method does not enable rapid diagnosis and is technically difficult. Moreover, viral culture does not necessarily correspond to HCMV disease. A reliable relation between virus isolation from peripheral leukocytes and the appearance of clinical symptoms may not exist in some immunosuppressed patients (Delgado, R. *et al.*, J Clin. Microbiol. 30, 1876-1878., 1992). Also urinary or pharyngeal shedding of the virus frequently occurs without clinical symptoms and organ involvement. Amplification of HCMV DNA in peripheral leukocytes by polymerase chain reaction (PCR), although a very sensitive technique for CMV viremia, is not usable as a marker of clinically symptomatic HCMV infection either. Due to the high sensitivity of enzymatic amplification, occasionally HCMV DNA was detectable in peripheral leukocytes without HCMV-related disease. Latent viral genomes may be detected by this technique or a patient may remain HCMV-DNA positive over a prolonged period of time after the disease has resolved (Jahn, G, *et al*., 1993; Zipeto, D. *et al*., J Clin. Microbiol. 30, 527-530., 1992; Delgado *et al*., 1992).

At the moment, the method of choice for the early diagnosis of acute symptomatic HCMV infection is the antigenemia assay based on immunological detection of the structural protein pp65 by using specific antibodies (Storch, G.A., *et al*., *J.* Clin. Microbiol. 32, 997-1003., 1994; Gerna, G. *et al*., J. Infect. Dis. 164, 488-498., 1991; Gerna, G., *et al*., *J* Clin. Microbiol. 30, 1232-1237.98., 1992). However, a matter of concern employing this method is its sensitivity. The number of pp65-positive cells in the early course of infection may be very low. Furthermore, in expressing cells stability of the pp65 antigen appeared to be limited (Chou, S., Curr. Opin. Infect. Dis. 5, 427-432., 1991) and sensitivity can be reduced due to the application of monoclonal antibodies rather than a pool of anti-pp65 antibodies that would recognize different epitopes of the protein.

Since viral replication requires transcription of mRNA species, the use of HCMV mRNA detection as a marker for active CMV infection was investigated (Bitsch, A. *et al*., J Infect. Dis 167, 740-743, 1993.
Recently, HCMV infections were examined on the transcript level using RNA amplification (Bitsch, A et al., J Infect. Dis 167, 740-743., 1993; Meyer, T.et al., Mol. Cell Probes. 8, 261-271., 1994; Gerna, G., et al., J Clin. Microbiol. 30, 1232-1237.98, 1993; Gerna, G., et al., J Clin. Microbiol. 30, 1232-1237.98., 1992).
*A primerset for the detection of a nucleic acid sequence in* the *late (LA) gene of cytomegalovirus DNA is disclosed in EP586011*. In principle, like detection of viral antigens, analysis of viral transcripts expressed in association with viral replication should allow reliable diagnosis of symptomatic infections.

The present invention provides a method for the detection of clinically symptomatic CMV disease based on the detection of late mRNA sequences of HCMV and nucleic acid sequences that could be used with said method.

Transcription of late mRNA species requires viral replication and, therefore, could be specific for active infection.

With the present invention it has been found that the detection of a certain late mRNA of HCMV is related to the appearance of clinical symptoms of HCMV disease. A method is provided for the diagnosis of HCMV disease characterized in that the presence of mRNA encoding a late structural protein of the human Cytomegalovirus in a blood sample of an individual suspected of carrying said disease is detected, said method comprising the following steps:
- amplifying a target sequence within said mRNA using a primer pair capable of specifically reacting with said target sequence and suitable amplification reagents,
- reacting the sample optionally containing amplified nucleic acid with a labeled nucleic acid probe having a sequence complementary to part of the amplified sequence and
- detecting hybrids formed between the amplified sequence and the probe.

When the presence or absence of certain late mRNAs in patients was correlated to their clinical status, a striking relation was observed between the presence of these late mRNAs and possibly CMV-related clinical symptoms.

The sensitivity and reliability of CMV mRNA detection is greatly dependent on primer selection, since there is sequence variation among strains of CMV potentially in every region of the genome. Ideally, primer selection should be based on knowledge of interstrain variability in candidate primer sequences and the consequences of mismatching at primer sites. (Chou S., J. of Clin. Microbiol., 2307-2310, 1992).

Therefore, the need exists for suitable oligonucleotides including nucleic acid sequences that can be used as primers and hybridization-probes for the amplification and subsequent detection of all strain variants of CMV.

The present invention is related to the detection of a certain late HCMV mRNA and provides suitable primers and probes for the amplification and subsequent detection of this mRNA. The binding sites of the primers and probes according to the present invention are located in the matrix tegument protein pp67 encoding gene sequence (UL65), expressed during the late phase of CMV infection.

The term "oligonucleotide" as used herein refers to a molecule comprised of two or more deoxyribonucleotides or ribonucleotides such as primers and probes.

The term "primer" as used herein refers to an oligonucleotide either naturally occurring (e.g. as a restriction fragment) or produced synthetically, which is capable of acting as a point of initiation of synthesis of a primer extension product which is complementary to a nucleic acid strand (template or target sequence) when placed under suitable conditions (e.g. buffer, salt, temperature and pH) in the presence of nucleotides and an agent for nucleic acid polymerization, such as DNA dependent or RNA dependent polymerase. A primer must be sufficiently long to prime the synthesis of extension products in the presence of an agent for polymerization. A typical primer contains at least about 10 nucleotides in length of a sequence substantially complementary (P1) or homologues (P2) to the target sequence, but somewhat longer primers are preferred. Usually primers contain about 15-26 nucleotides but longer primers, up to 35 nucleotides may also be employed.

Normally a set of primers will consist of at least two primers, one 'upstream' and one 'downstream' primer which together define the amplificate (the sequence that will be amplified using said primers).

An upstream primer (P1) will always contain a sequence substantially complementary to the target sequence to which it may anneal. A downstream primer (P2) will contain a sequence substantially homologues to the target sequence.

A primer may, optionally, also comprise a promoter sequence. The term "promoter sequence" defines a region of a nucleic acid sequence that is specifically recognized by an RNA polymerase that binds to a recognized sequence and initiates the process of transcription by which an RNA transcript is produced. In principle any promoter sequence may be employed for which there is a known and available polymerase that is capable of recognizing the initiation sequence. Known and useful promoters are those that are recognized by certain bacteriophage RNA polymerases such as bacteriophage T3, T7 or SP6.

It is understood that a nucleic acid primer consisting of the sequences of the present invention may contain minor deletions, additions and/or substitutions of nucleic acid bases, to the extent that such alterations do not negatively affect the yield or product obtained to a significant degree.

Various techniques for amplifying nucleic acid are known in the art. One example of a technique for the amplification of a DNA target segment is the so-called "polymerase chain reaction" (PCR). With the PCR technique the copy number of a particular target segment is increased exponentially with a number of cycles. In each cycle a DNA primer is annealed to the 3' side of each strand of the double stranded DNA-target sequence. The primers are extended with a DNA polymerase in the presence of the various mononucleotides. The extension products are rendered single stranded by thermal denaturation and each strand can serve as a template for primer annealing and subsequent elongation in a following cycle. The PCR method has been described in Saiki *et al*., Science 230, 135, 1985 and in European Patents no. EP 200362 and EP 201184.

Another technique for the amplification of nucleic acid is the so-called transcription based amplification system (TAS). TAS employs an RNA-transcript-production step from a DNA, synthesized to incorporate a segment of the target sequence and a promoter, to enable transcription from the segment of a RNA with the sequence complementary to that of the target. Multiple cycles can be carried out as the RNA made in the transcription step can serve as template for making similarly transcribable DNA, which in turn, can be transcribed to yield additional RNA. The TAS method is described in International Patent Appl. no. WO 88/10315.

Yet another method for the amplification of nucleic acid is the nucleic acid sequence based amplification process ("NASBA") as described in European Patent no. EP 329822. Like TAS, NASBA includes a RNA-transcript production step using T7 RNA polymerase to transcribe multiple copies of RNA from a double stranded DNA template including a T7 promoter sequence.

An oligonucleotide sequence used as detection-probe may be labeled with a detectable moiety. Various labeling moieties are known in the art. Said moiety may, for example, either be a radioactive compound, a detectable enzyme (e.g. horse radish peroxidase (HRP)) or any other moiety capable of generating a detectable signal such as a colorimetric, fluorescent, chemiluminescent or electrochemiluminescent signal.

For the pp67 gene sequence interstrain variations between CMV AD169 and Towne exist. Therefore, for this target two primer pairs were chosen, CMV-pp67-1 and CMV-pp67-4 (Table 1), either of which was derived from the same region of the gene but each based on a different laboratory strain. An example of oligonucleotides for the detection of pp67 mRNA are oligonucleotides, 10-35 nucleotides in length comprising, at least a fragment of 10 nucleotides, of a sequence selected from the group consisting of: and

A preferred primer set according to the present invention includes the following oligonucleotide sequences: or in combination with 5'-GACCTGATATCCCTCCATATA-3'. The T7 promoter sequence is shown, but may be replaced by any other suitable promoter sequence.

A probe that may be used for the detection of the amplificate generated using this primer set may comprise an oligonucleotide consisting essentially of the following sequence: 5'-GGATTCGGACTTTCCGTTCGA-3'.

Probes comprising said sequence are also part of the present invention.

For RNA amplification (as with the method according to the invention), the NASBA technology, or another transcription based amplification technique, is a preferred technology. This amplification method consists of an in vitro primer-directed amplification of a specific RNA region (Kievits *et al*., 1991; Compton J., Nature 350, 91-92, 1991).

If RT-PCR is used for the detection of viral transcripts differentiation of mRNA- and DNA-derived PCR products is necessary. For spliced transcripts, like the IEA mRNA, the exon-intron structure can be used. However, mRNA species encoding the late structural proteins are almost exclusively encoded by unspliced transcripts. DNAse treatment prior to RT-PCR can be employed (Bitsch, A. *et al*., *J* Infect. Dis 167, 740-743., 1993; Meyer, T. *et al*., Mol. Cell Probes. 8, 261-271., 1994), but sometimes fails to remove contaminating DNA sufficiently (Bitsch, A. *et al*., 1993).

In contrast to RT-PCR, NASBA, which is based on RNA transcription by T7 RNA polymerase (Kievits *et al*., 1991; Compton, 1991), does not need differentiation between RNA- and DNA-derived amplification products since it only uses RNA as its principal target. NASBA enables specific amplification of RNA targets even in a background of DNA. Especially for unspliced targets like almost all late HCMV gene transcripts, this method is beneficial as it circumvents DNAse treatment which occasionally might be inefficient (Bitsch, A. *et al*., 1993).

This method was used for the analysis of CMV transcripts in whole blood samples from transplant recipients and HIV-infected individuals.

Test kits for the detection of CMV in clinical samples are also part of the present invention. A test kit according to the invention may comprise a set of primers according to the invention and a probe according to the invention. Such a test kit may additionally comprise suitable amplification reagents such as DNA and or RNA polymerases and mononucleotides. Test kits that can be used with the method according to the invention may comprise the primers and probe according to the invention for the amplification and subsequent detection of pp67 mRNA.

The invention is further exemplified by the following example.

### EXAMPLES:

### Example 1: Analysis of CMV DNA and mRNA in clinical samples.

### MATERIALS AND METHODS

### Clinical Specimens

Samples from patients clinically at risk of infection with CMV were analyzed for the presence of mRNAs encoding the immediate early antigen (IEA) or the matrix tegument protein pp67 expressed during the late phase of CMV infection.

The thirty-five blood samples were obtained from immunocompromised patients including 22 heart, liver, or kidney transplant recipients, 8 AIDS patients, two patients with leukemia, one patient with a myelodysplastic syndrome, one patient with primary Epstein Barr virus mononucleosis, and one patient with Kaposi sarcoma. Ethylene diaminotetraacetic acid (EDTA) anticoagulated blood samples submitted consecutively as received by the laboratory, were mixed with nine volumes of Lysis buffer [50 mM Tris-Hydrochloric acid (pH 6.4); 20 mM EDTA; 1.3% (w/v) Triton X-100; 5.25 M Guanidinium thiocyanate] and stored at -70°C until use.

### Nucleic acid isolation

From the anticoagulant-treated blood specimens total nucleic acid was isolated using guanidinium thiocyanate-mediated cell lysis and adsorption of nucleic acid to silica particles (Boom *et al*., J. of Clin. Microbiol. 28, 495-503, 1990).

Whole blood samples in Lysis buffer were thawed and from each sample 1 ml (equivalent to 100 *µ*l whole blood) was transferred into an Eppendorf tube. Subsequently, 70 *µ*l of Hydrochloric acid-activated silicum dioxide particles [size-selected suspension of 1 mg/ml in 0.1 M Hydrochloric acid (Sigma); see ref. Boom et al., 1990] were added and the suspension was incubated during 10 minutes at room temperature with regular vortexing. Nucleic acid bound to the silica was spun down by centrifugation. Pelleted silica particles were washed twice with 1 ml GuSCN wash buffer [50 mM Tris-Hydrochloric acid (pH 6.4); 5.25 M Guanidinium thiocyanate], followed by two washing steps with 1 ml 70% ethanol and a single washing step with 1 ml acetone. After each washing step, the suspension was briefly centrifuged and the silica pellet was resuspended in the next washing solution by thorough mixing. After removal of the acetone, the silica particles were dried by incubation at 56°C in a heating block during 10 minutes. Nucleic acid was eluted from the silica particles by incubation in 100 *µ* l distilled water at 56°C during 10 minutes. Finally, the silica particles were spun down again and the supernatant was carefully pipetted into fresh reaction tubes avoiding any carry-over of silica. Extracted nucleic acid samples were stored at -70°C until use.

Prior to the detection of CMV mRNAs in these isolates, the integrity and amount of extracted RNA was validated.

Therefore, samples were analyzed for the presence of U1 snRNP-specific A protein (U1A) mRNA (Sillekens, P.T.G., *et al*., EMBO J. 6, 3841-3848., 1987), being a relatively low abundant message, transcribed from a cellular housekeeping gene. As revealed by Northern blot analysis, presence of amplifiable U1A mRNA was obvious in all samples (data not shown).

### Primers and probes

Sequences and polarity of the primers and of the probes used for specific detection, are shown in Table 1.

All oligonucleotide primers and probes were synthesized on a PCR-MATE 391 DNA synthesizer (Applied Biosystems) using phosphoramidite biochemistry. Oligonucleotides for ELGA detection (see below) were synthesized with a 5'-amino link (Aminolink 2; Applied Biosystems) for subsequent coupling of Horse Radish Peroxidase (HRP).

Amplification primers were purified by electrophoretically separating the crude oligonucleotide solutions over a 20% polyacrylamide/7M Urea slabgel and subsequent elution of the full-length oligonucleotide from the corresponding gel band. After elution from the gel slices and concentration by ethanol precipitation, primers were dissolved in Milli-Q water and concentrations determined by OD(260 nm) measurement.

Detection probes were conjugated with HRP (Boehringer) by coupling the enzyme to the amino link of the oligonucleotide using the cross-linking reagents SDPD (Pharmacia) and EMCS (Fluka). Unbound HRP was removed over a Qiagen Tip-100 column (Qiagen). The HRP-labeled oligonucleotides were purified by polyacrylamide gel electrophoresis and subsequent elution of the HRP-oligonucleotides from the gel slices by overnight incubation in water. The amount of HRP-conjugated oligonucleotide was calculated from OD(260nm) and OD(400 nm) measurement. The solutions were stored at -70°C.

### NASBA amplification

RNA-amplifications were performed using NASBA, since this amplification technology is capable of specifically amplifying RNA in a background of DNA. These amplification reactions were carried out using a standard NASBA protocol:

To set up an amplification reaction, 10*µ*l of *2.5* x reaction buffer [100mM Tris-Hydrochloric acid (pH 8.5); 30mM Magnesium chloride; 105mM Potassium chloride; 12.5mM Dithriothreitol; 2.5 mM of each of dNTP; 5mM of ATP, CTP and UTP; 3.75mM of GTP; 1.25mM of ITP] was added to a reaction tube together with 6.25*µ*l 4 x primermix [0.8*µ*M of each primer; 60% Dimethylsulphoxide], 5*µ*l nucleic acid solution, and 1.75 *µ*l distilled water. This mixture was heated at 65 °C during 5 minutes, after which the tubes were placed at 41°C. Two *µ*l of enzyme mix [40 units T7 RNA polymerase; 8 units AMV reverse transcriptase; 0.1 unit RNase H; 1.25 *µ*g/*µ*l BSA] were added and the contents of the tube was mixed by gentle tapping. The reaction was incubated at 41 °C for 90 minutes and stopped by placing it at -20°C.

### Polymerase Chain Reaction

For the detection of the corresponding genes of the HCMV mRNAs PCR amplification was used (Performed essentially as described in Saiki *et al*, 1985).

As template DNA, 5 *µ*l nucleic acid solution were added to a total of 20 *µ*l of reaction mixture for amplification containing the appropriate primer pair (15 pmol each), deoxyribonucleoside triphosphates (200 *µ*M each; Pharmacia), 2 *µ*l of 10 x PCR buffer (Perkin-Elmer), and 1.25 units Taq polymerase. Reactions were overlaid with 100 *µ*l of mineral oil to prevent evaporation. The amplification was performed in a DNA thermal cycler (Perkin-Elmer) by 40 cycles of denaturation at 94°C for 1 min, primer annealing at 60°C for 1 min, chain extension at 72°C for 2 min, and a final extension segment at 72°C for 10 min.

### Southern blot analysis of PCR-amplified products

Amplified DNA was transferred from a 2.0 % pronarose gel (Hispanagar, S.A.) to a nylon membrane (Zeta-probe; BioRad, USA) by vacuum blotting in 2 x SSC [1 x SSC is 150 mM Sodium chloride; 15 mM Sodium citrate] during 2 hours. Membranes were preincubated at 50°C in a hybridization solution [0.5 M Sodium phosphate (pH 7.2); 7% Sodium dodecyl sulphate] during 30 minutes prior to the addition of ³²P labeled oligonucleotide probe to a final concentration of about 10⁵ cpm/ml. Hybridization was performed overnight at 50 °C. Subsequent washings were carried out at 50 °C in 0.3 x SSC supplemented with 0.1 % SDS. Autoradiography was performed for several hours at -70 °C with Kodak Royal X-omat film and intensifying screens.

### Analysis of NASBA-amplified products (ELGA)

For the analysis of NASBA products a non-radio-active enzyme linked gel assay (ELGA) based on liquid hybridization was used.

Hybridization of amplification product to a specific HRP labeled oligonucleotide probe was performed by mixing 2*µ* l of a amplification reaction with 1 *µ*l 5 x SSC, 1*µ*l concentrated loading buffer [25% (v/v) Glycerol; 10mM Sodium phosphate buffer (pH 7.0); 0.05% bromophenol blue; 0.01% Xylene cyanol], and 1*µ*l HRP-labeled oligonucleotide solution containing about 10¹⁰ molecules per *µ*l, followed by incubation at 45 °C during 15 minutes. After hybridization, half of the reaction mixture was directly applied onto a 7% polyacrylamide gel supplemented with 0.04% (w/v) dextrane sulphate. After separation of bound and unbound HRP-labeled oligonucleotide by electrophoresis, the probe was visualized in the gel by direct staining with 50 ml substrate solution [125*µ*g 3,3',5,5'-tetramethylbenzidine per ml; 0.003 % Hydrogen peroxide; 100mM Sodium citrate buffer (pH 5.2)] for about 10 minutes at room temperature. Finally, the gel was fixed by overnight incubation in a 50% (v/v) methanol solution and air dried.

### RESULTS:

### Primers and sensitivity

To determine the analytical sensitivity attainable in NASBA with the primer pairs a standard dilution series of in vitro generated CMV RNA was evaluated. An RNA template of known concentration was generated in vitro from a cloned subfragment of the CMV AD 169 EcoRI fragment J (Schrier, R.D., *et al*., Science 230, 1048-1051., 1985) for IEA and a CMV clone encompassing the pp67 gene for pp67. Standard dilution series were prepared from the in vitro generated RNA.

Sensitivity of the CMV-IEA (E4) primer set was reproducibly found to be at least 100 molecules of in vitro generated RNA input in NASBA reaction. For the CMV-IEA(E2/E3) primer pair a comparable sensitivity of 100 molecules could be achieved.

Primer performance of the pp67 primer pairs was evaluated on in vitro generated RNA transcribed from a cloned fragment of the pp67 gene of CMV AD169. For the CMV-pp67-4 primer pair, derived from AD169, NASBA conditions could be optimized such that the sensitivity of this primer pair was 100 molecules of in vitro generated RNA. As could be anticipated from the mismatches of the downstream primer of CMV-pp67-1, based on the Towne strain sequence, no amplification product could be generated with this primer pair from the CMV AD169-derived pp67 RNA.

To establish whether in addition to in vitro generated RNA also the genuine viral mRNAs could be identified by these primer sets, total nucleic acid from fibroblast cells infected with CMV AD 169 was extracted and amplified by NASBA. All primer sets revealed hybridization signals on Northern blot that correspond to CMV specific RNA derived amplification products. Northern blot analysis was performed as described below.

Based on ten-fold dilution series of the recombinant plasmids used for in vitro transcription of IEA RNA or pp67 RNA, the lower detection limit of the NASBA primer pairs, when used in PCR, was about 50-100 genome equivalents.

### Detection of HCMV mRNA and DNA.

Because the internal U1A mRNA control was positive in all samples, the entire series of 35 specimens was further analyzed for the presence of the IEA gene and its corresponding mRNA. When amplification by PCR was performed, eighteen samples were positive for CMV-DNA when using the IEA gene primers (table 2). However, PCR amplification with the pp67 gene primers failed to detect CMV-DNA in two of these eighteen samples (OT28 and OT34, Table 2). This indicates that despite the fact that already two primer pairs were used for the pp67 gene, still two samples were falsely negative for CMV DNA. Most likely, this is due to sequence variation among the clinical strains in this part of the HCMV genome, since the sensitivity of the primer sets for the IEA gene target and the pp67 gene target are comparable.

When the samples were analyzed for IEA mRNA by NASBA amplification with the same primer pairs as used for DNA detection by PCR, essentially all samples that were positive by PCR were also found positive by NASBA. Therefore with the exception of a single patient sample in all specimens positive for CMV-IEA DNA the cognate mRNA could be detected as well.

Analysis for pp67 mRNA by NASBA revealed a strikingly different result.

In contrast to IEA for which, with the exception of a single sample, all DNA-positive samples were positive for IEA-mRNA as well, pp67 mRNA could only be detected in a subset of the CMV-DNA positive patients (Table 2). In Table 3, the data for mRNA and DNA detection for the IEA target and the pp67 target are summarized. When the presence or absence of pp67 mRNA in these patients was correlated to their clinical status, a striking relation was observed between the presence of pp67 mRNA and possibly CMV-related clinical symptoms (Table 4).Two patients in this group showed symptoms of transplant rejection with graft dysfunction and fever. Clinical diagnoses in other patients were gastritis after heart transplantation and retinitis in a kidney transplant recipient. Retinitis was further observed in three CMV-pp67 mRNA positive AIDS patients, two of which also suffered from esophagitis.

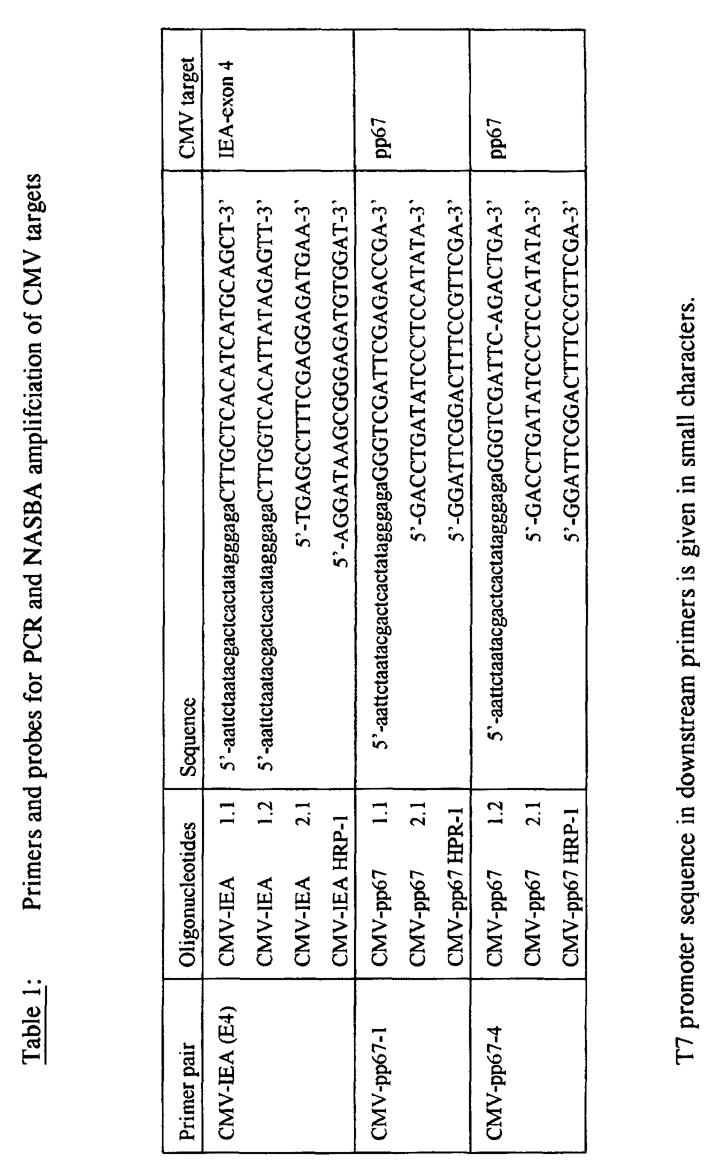

**Table 2:**

| Analysis of CMV-IEA and CMV-pp67 DNA and mRNA in clinical specimens | | | | |
|---|---|---|---|---|
| No. | IEA-DNA (PCR) | IEA-mRNA (NASBA) | pp67-DNA (PCR) | pp67-mRNA (NASBA) |
| 0T01 | - | - | - | - |
| 0T02 | ++ | ++ | ++ | ++ |
| 0T03 | - | - | - | - |
| 0T04 | ++ | (+) | ++ | ++ |
| 0T06 | - | - | - | - |
| 0T07 | + | + | (+) | - |
| 0T09 | + | ++ | (+) | - |
| 0T10 | - | - | - | - |
| 0T11 | - | - | - | - |
| 0T12 | ++ | ++ | ++ | (+) |
| 0T13 | ++ | ++ | ++ | (+) |
| 0T14 | ++ | ++ | ++ | ++ |
| 0T16 | - | - | - | - |
| 0T17 | ++ | ++ | ++ | (+) |
| 0T18 | - | - | - | - |
| 0T19 | ++ | ++ | (+) | - |
| 0T20 | - | - | - | - |
| 0T21 | - | - | - | - |
| 0T22 | ++ | ++ | ++ | ++ |
| 0T23 | - | - | - | - |
| 0T24 | ++ | ++ | ++ | ++ |
| 0T26 | ++ | ++ | + | - |
| 0T27 | - | - | - | - |
| 0T28 | (+) | ++ | - | - |
| 0T29 | ++ | ++ | ++ | - |
| 0T31 | ++ | ++ | ++ | ++ |
| 0T32 | - | - | - | - |
| 0T33 | ++ | + | ++ | ++ |
| 0T34 | (+) | - | - | - |
| 0T35 | - | - | - | - |
| 0T36 | - | - | - | - |
| 0T37 | - | - | - | - |
| 0T38 | - | - | - | - |
| 0T39 | - | - | - | - |
| 0T40 | (+) | ++ | (+) | - |
| No. patient sample number + positive | | | | |
| ++ strongly positive (+) weakly positive | | | | |

**Table 3**

| CMV target | DNA-pos/RNA-pos | DNA-pos/RNA-neg | DNA-neg/RNA-neg |
|---|---|---|---|
| IEA (n = 35) | 17 | 1 | 17 |
| pp67 (n= 35) | 9 | 7 | 19 |
| * Two samples negative for pp67 DNA and mRNA while positive for IEA DNA and mRNA. | | | |
| * A number of CMV-positive samples does not contain detectable levels of pp67 mRNA. | | | |

**Table 4:**

| Correlation between presence of HCMV pp67 mRNA and clinical status. | |
|---|---|
| A. pp67 DNA-pos/RNA-pos patients | |
| Patient | Clinical status |
| Heart transplantation | graft rejection, fever, bronchial carcinoma, |
| Kidney transplantation | immunocytoma |
| Kidney transplantation | retinitis, fever |
| Kidney transplantation | graf rejection, fever |
| AIDS | |
| AIDS | retinitis, lobular hepatitis |
| AIDS | retinitis, esophagitis |
| Heart transplantation | retinits, esophagitis |
| AIDS | gastritis |
| | epilepsy |

| B. pp67 DNA-pos/RNA-neg patients | |
|---|---|
| Patient | Clinical status |
| AIDS | Kaposi sarcoma, cryptococcus meningitis |
| AIDS | |
| Kidney transplantation | |
| Kidney transplantation | |
| Heart transplantation | |
| Heart transplantation | secundary EBV infection, immunocytoma |
| Heart transplantatin | |

| C. pp67 DNA-neg/RNA-neg patients | |
|---|---|
| Patient | Clinical status |
| Leukemia | |
| Liver transplantation | elevated liver enzymes |
| Heart transplantation | graft rejection |
| Heart transplantation | graft rejection |
| Heart transplantation | |
| AIDS | lobular pneumonia, candida infection |
| -- | acute myeloid leukemia, vasculitis |
| Heart transplantation | |
| -- | primary EBV mononucleosis |
| Kidney transplantation | |
| AIDS | |
| Heart transplantation | graft rejection |
| AIDS | |
| Kidney transplantation | tuberculosis |
| -- | myelodysplastic syndrome |
| Heart transplantation | seisure |
| Heart transplantation | sternum infection, staphylococcus aureas |
| Heart transplantation | |
| Heart transplantation | |

## Claims

1. Method for the diagnosis of symptomatic CMV disease, **characterized in that** the presence of mRNA encoding the late structural protein pp 67 of the human Cytomegalovirus in a blood sample *taken from* an individual, suspected of carrying said disease, is detected *in vitro,* said method comprising the following steps:
- amplifying a target sequence within said mRNA using a primer pair capable of specifically reacting with said target sequence and suitable amplification reagents, said primer pair being designed to accommodate interstrain variation,
- reacting the sample, optionally containing amplified nucleic acid, with a labeled nucleic acid probe having a sequence complementary to part of the target sequence
- detecting hybrids formed between the target sequence and the probe.

2. Method according to claim 1 wherein the mRNA is amplified, using a transcription based amplification technique.

3. Method according to claim 2, wherein said amplification technique is NASBA.

4. Oligonucleotide, corresponding to part of a nucleic acid sequence encoding HCMV pp67, said oligonucleotides being 10-35 nucleotides in length and comprising, at least a fragment of 10 nucleotides, of a sequence selected from the group consisting of: or its complementary sequence.

5. Oligonucleotide according to claim 4 linked to a promoter sequence.

6. Set of primers, for the amplification of a target sequence located within a HCMV pp67 sequence, comprising one primer consisting essentially of the nucleic acid sequence: linked to a promoter sequence and a second primer consisting essentially of the nucleic acid sequence 5'-GACCTGATATCCCTCCATATA-3'.

7. Set of primers, for the amplification of a target sequence located within HCMV pp67 sequence, comprising one primer consisting essentially of the nucleic acid sequence: linked to a promoter sequence and a second primer consisting essentially of the nucleic acid sequence 5'-GACCTGATATCCCTCCATATA-3'. essentially of the nucleic acid sequence 5'-GACCTGATATCCCTCCATATA-3'.

8. Use of any of the oligonucleotides according to claim 4 as a primer in the method of claim 2.

9. Use of an oligonucleotide consisting essentially of the sequence 5'-GGATTCGGACTTTCCGTTCGA-3', provided with a detectable label, as a probe in the method of claim 1.

10. Test kit for the diagnosis of HCMV disease comprising:
- one or more set(s) of primers according to claim 6 and /or 7,
- an oligonucleotide comprising a nucleic acid sequence complementary to at least part of the amplified nucleic acid sequence defined by said set of primers, provided with a detectable label.

## Patentansprüche

1. Verfahren zur Diagnose von symptomatischer CMV-Erkrankung **dadurch gekennzeichnet, dass** das Vorhandensein von mRNA, die das späte strukturelle Protein des humanen Cytomegalovirus PP67 kodiert, in einer Blutprobe eines Individuums, das unter Verdacht steht die besagte Krankheit zu besitzen, in vitro nachgewiesen wird, wobei die besagte Methode die folgenden Schritte umfasst:
- Amplifizieren einer Zielsequenz innerhalb besagter mRNA mit Hilfe eines Primerpaars, das fähig ist spezifisch mit besagter Sequenz zu reagieren, und Amplifikationsreagenzien, wobei das besagte Primerpaar so gestaltet ist, um sich an Variationen zwischen den Stämmen anzupassen.
- Reagieren der Probe, die gegebenenfalls eine amplifizierte Nukleinsäure enthält, mit einer markierten Nukleinsäuresonde, die eine zu einem Teil der Zielsequenz komplementäre Sequenz besitzt, und
- Nachweisen von Hybriden, die sich zwischen der Sequenz und der Sonde gebildet haben.

2. Verfahren gemäss Anspruch 1, worin die mRNA mit Hilfe einer Transkriptions-basierenden Amplifikationsmethode amplifiziert wird.

3. Verfahren gemäss Anspruch 2, worin die besagte Amplifikationsmethode NASBA ist.

4. Oligonukleotid, welches einem Teil der HCMV pp67 kodierenden Nukleinsäuresequenz entspricht, wobei die besagten Oligonukleotide 10-35 Nukleotide in der Länge aufweisen und wenigstens ein Fragment von 10 Nukleotiden einer Sequenz umfassen, die ausgewählt ist aus der Gruppe bestehend aus: oder dessen komplementäre Sequenz.

5. Oligonukleotid gemäss Anspruch 4 verknüpft an eine Promotorsequenz.

6. Primersatz zur Amplifikation einer Zielsequenz, die innerhalb einer HCMV pp67 Sequenz lokalisiert ist, umfassend einen Primer, der im wesentlichen aus der Nukleinsäuresequenz: verknüpft an eine Promotorsequenz besteht und einem zweiten Primer, der im wesentlichen aus der Nukleinsäuresequenz 5'-GACCTGATATCCCTCCATATA-3' besteht.

7. Primersatz zur Amplifikation einer Zielsequenz, die innerhalb einer HCMV pp67 Sequenz lokalisiert ist, umfassend einen Primer, der im wesentlichen aus der Nukleinsäuresequenz: verknüpft an eine Promotorsequenz besteht und einem zweiten Primer, der im wesentlichen aus der Nukleinsäuresequenz 5'-GACCTGATATCCCTCCATATA-3' besteht.

8. Verwendung jeglicher Oligonukleotide gemäss Anspruch 4 als Primer in der Methode gemäss Anspruch 2.

9. Verwendung eines Oligonukleotides, das im wesentlichen aus der Sequenz 5'-GGATTCGGACTTTCCGTTCGA-3' besteht und mit einem nachweisbaren Marker versehen ist, als Sonde in der Methode gemäss Anspruch 1.

10. Testkit zur Diagnose von HCMV-Erkrankung umfassend:
- ein oder mehrere Primerset(s) gemäss Anspruch 6 und/oder 7,
- ein Oligonukleotid umfassend eine Nulcleinsäurensequenz, die zu mindestens einem Teil der amplifizierten Nukleinsäurensequenz, die durch das besagte Primerset definiert ist, komplementär ist, und mit einem nachweisbaren Marker versehen ist.

## Revendications

1. Procédé pour diagnostiquer une maladie CMV symptomatique, **caractérisé en ce que** la présence d'ARNm codant pour la protéine structurale tardive pp67 du Cytomcgalovirus humain dans un échantillon sanguin prélevé d'un individu, suspecté d'être atteint par ladite maladie, est détectée *in vitro,* ledit procédé comprenant les étapes suivantes :
- l'amplification de la séquence cible dudit ARNm en utilisant une paire d'amorces capable de réagir spécifiquement avec ladite séquence cible et des réactifs d'amplification appropriés, ladite paire d'amorces étant conçue pour s'adapter à la variation existant entre les souches,
- la réaction de l'échantillon, le cas échéant contenant un acide nucléique amplifié, avec une sonde d'acide nucléique marqué ayant une séquence complémentaire à une partie de la séquence cible,
- la détection d'hybrides formés entre la séquence cible et la sonde.

2. Procédé selon la revendication 1 dans lequel l'ARNm est amplifié, en utilisant une technique d'amplification basée sur la transcription.

3. Procédé selon la revcndication 2, dans lequel ladite technique d'amplification est NASBA.

4. Oligonucléotide, correspondant à une partic de la séquence d'acide nucléique codant pour la pp67 de HCMV, lesdits oligonucléotides étant longs de 10 à 35 nucléotides et comprenant au moins un fragment de 10 nucléotides d'une séquence choisie parmi le groupe consistant en : ou sa séquence complémentaire.

5. Oligonucléotide selon la revendication 4 rattaché à une séquence de promoteur.

6. Jeu d'amorces, destiné à l'amplification d'une séquence cible située dans une séquence de pp67 de HCMV, comprenant une amorce consistant essentiellement en la séquence d'acide nucléique : rattachée à une séquence de promoteur et à une seconde amorce consistant essentiellement en la séquence d'acide nucléique

7. Jeu d'amorces, destiné à l'amplification d'une séquence cible située dans la séquence de pp67 de HCMV, comprenant une amorce consistant essentiellement en la séquence d'acide nucléique : rattachée à une séquence de promoteur et une seconde amorce consistant essentiellement en la séquence d'acide nucléique

8. Utilisation de l'un des oligonucléotides selon la revendication 4 comme amorce dans le procédé de la revendication 2.

9. Utilisation de l'un des oligonucléotides consistant essentiellement en la séquence 5'-GGATTCGGACTTTCCGTTCGA-3', muni d'un marqueur détectable, tel qu'une sonde du procédé selon la revendication 1.

10. Kit de test pour diagnostiquer la maladie de HCMV comprenant :
- un ou plusieurs jeu(x) d'amorces selon la revendication 6, et/ou 7,
- un oligonucléotide comprenant une séquence d'acide nucléique complémentaire à au moins une partie de la séquence d'acide nucléique amplifiée définie par ledit jeu d'amorces, muni d'un marqueur détectable.


### SEQUENCE LISTING

(1) GENERAL INFORMATION:
   (i) APPLICANT:
      (A) NAME: Akzo Nobel N.V.
      (B) STREET: Velperweg 76
      (C) CITY: Arnhem
      (E) COUNTRY: The Netherlands
      (F) POSTAL CODE (ZIP): 6824 BM
   (ii) TITLE OF INVENTION: Primers and probes for the detection of CMV nucleic acid.
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: EP 94202360.7
      (B) FILING DATE: 18-AUG-1994
(2) INFORMATION FOR SEQ ID NO: 1:
   (i) SEQUENCE CHARACTERISTICS:
      (A) LENGTH: 48 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 1:
   AATTCTAATA CGACTCACTA TAGGGAGACT TGCTCACATC ATGCAGCT 48
(2) INFORMATION FOR SEQ ID NO: 2:
   (i) SEQUENCE CHARACTERISTICS:
      (A) LENGTH: 48 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 2:
   AATTCTAATA CGACTCACTA TAGGGAGACT TGGTCACATT ATAGAGTT 48
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 3:
   TGAGCCTTTC GAGGAGATGA A 21
(2) INFORMATION FOR SEQ ID NO: 4:
   (i) SEQUENCE CHARACTERISTICS:
      (A) LENGTH: 22 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 4:
   AGGATAAGCG GGAGATGTGG AT 22
(2) INFORMATION FOR SEQ ID NO: 5:
   (i) SEQUENCE CHARACTERISTICS:
      (A) LENGTH: 46 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 5:
   AATTCTAATA CGACTCACTA TAGGGAGAGG GTCGATTCGA GACCGA 46
(2) INFORMATION FOR SEQ ID NO: 6:
   (i) SEQUENCE CHARACTERISTICS:
      (A) LENGTH: 18 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 6:
   GGGTCGATTC GAGACCGA 18
(2) INFORMATION FOR SEQ ID NO: 7:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 7:
   GACCTGATAT CCCTCCATAT A 21
(2) INFORMATION FOR SEQ ID NO: 8:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 8:
   GGATTCGGAC TTTCCGTTCG A 21
(2) INFORMATION FOR SEQ ID NO: 9:
   (i) SEQUENCE CHARACTERISTICS:
      (A) LENGTH: 45 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 9:
   AATTCTAATA CGACTCACTA TAGGGAGAGG GTCGATTCAG ACTGA 45
(2) INFORMATION FOR SEQ ID NO: 10:
   (i) SEQUENCE CHARACTERISTICS:
      (A) LENGTH: 17 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 10:
   GGGTCGATTC AGACTGA 17
(2) INFORMATION FOR SEQ ID NO: 11:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 11:
   GACCTGATAT CCCTCCATAT A 21
(2) INFORMATION FOR SEQ ID NO: 12:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA to mRNA
   (xi) SEQUENCE DESCRIPTION: SEQ ID NO: 12:
   GGATTCGGAC TTTCCGTTCG A 21